# EUROPEAN PATENT APPLICATION

(11) **EP 4 825 491 A1**
(43) Date of publication of application: **21.10.2026**
(21) Application number: 25171323.6
(22) Date of filing: 17.04.2025
(51) Int. Cl.: B01L 3/00

(54) **DIGITAL MICROFLUIDICS CARTRIDGE WITH INTEGRATED TIRF DETECTION**

(71) Applicant: Eviti Nordic AB, 427 34 Billdal (SE)
(72) Inventor: Kaminski, Tim Patrick, Billdal (SE); Kleinboelting, Silke, Lindome (SE)
(74) Representative: dompatent

(57) **Abstract**

This invention relates to a device to detect and/or quantify at least one biomarker or at least one ligand in a fluid, comprising a digital microfluidics (DMF) platform, at least one detection area, a lens or an objective, and a light source, characterized in that the DMF platform comprises a cartridge with two opposite panels, whereas both panels are conductive or made conductive, and in the detection area at least one of the panels can be functionalized to detect the at least one biomarker and, a lens or an objective is positioned to detect the signal of the at least one detection areas and, the light source is selected from LED, laser, arc lamps, gas discharge lamps or halogen lamps is provided. Both panels can optionally be conductive or made conductive, facilitating the manipulation of fluids through the application of electric fields. The DMF platform may feature at least one detection area, which serves as a zone for potential interaction with the fluid under study.

## Description

The present invention relates to a device to detect and/or quantify at least one biomarker or at least one ligand in a fluid, comprising a digital microfluidics (DMF) platform, at least one detection area, a lens or an objective, and a light source, characterized in that the DMF platform comprises a cartridge with two opposite panels, whereas both panels are conductive or made conductive, and in the detection area at least one of the panels can be functionalized to detect the at least one biomarker and, a lens or an objective is positioned to detect the signal of the at least one detection areas and, the light source is selected from LED, laser, arc lamp, gas discharge lamps or halogen lamps.

The detection and quantification of biomarkers and ligands in fluids is a critical process in medical diagnostics, environmental monitoring, and biotechnological research. Ensuring accurate and efficient measurement of these substances is essential for disease management, therapeutic monitoring, and various analytical applications.

Evanescent fields is a well known method to assess the binding of analytes to a sensor surface. They provide the advantage that they limit the detection volume to being close to the surface.

As the demand for accurate, rapid, and versatile biomarker detection continues to grow, especially in fields such as medical diagnostics and environmental monitoring, there is a high demand for innovative devices that can overcome these traditional barriers, offering enhanced performance and flexibility without compromising reliability or ease of use.

The objective technical problem underlying this invention is thus to provide a device to detect and/or quantify biomarkers or ligands in fluids that offers significant advantages in flexibility, analytical sensitivity, reduction of nonspecific background signals, multiplexing capacity, and robustness across various analytical, diagnostic, reduction of production costs, or bioassay applications.

In a first embodiment a device to detect and/or quantify at least one biomarker or at least one ligand in a fluid, comprising a digital microfluidics (DMF) platform, at least one detection area, a lens or an objective, and a light source, characterized in that the DMF platform comprises a cartridge with two opposite panels, whereas both panels are conductive or made conductive, and in the detection area at least one of the panels can be functionalized to detect the at least one biomarker and, a lens or an objective is positioned to detect the signal of the at least one detection areas and, the light source is selected from LED, laser, arc lamps, gas discharge lamps or halogen lamps is provided. Both panels can optionally be conductive or made conductive, facilitating the manipulation of fluids through the application of electric fields. The DMF platform may feature at least one detection area, which serves as a zone for potential interaction with the fluid under study.

Fluid within the meaning of the present invention can be human or animal body fluids such as blood, sweat, saliva, lymph fluid, urine etc. These can also be used diluted in a suitable buffer. Furthermore, the fluid can be a buffered or non-buffered solution of chemical compounds, or biological compounds such as antibodies, proteins, aptamers, peptides, nucleic acids or a combination thereof.

One panel within the detection area can be functionalized to act as a reactive surface where specific biomarkers or ligands might bind. This functionalization could involve coating the surface with biomolecules or chemical agents tailored to capture the desired targets within the fluid, enabling specific interactions desirable for the detection and quantification of the biomarker or ligand.

Additionally, the design may integrate optical elements like a lens or an objective, strategically positioned to collect signals from the detection area. This optical setup can enhance signal clarity and precision in measurement by using light-based detection methods to evaluate the presence and quantity of the target biomarker or ligand. The accurate focusing of light on the detection area in combination with detectors that are able to detect small signals or enhance the detected signals enables the capture and interpretation of subtle signals. In one embodiment the medium between the lens and the side panel has a lower refractive index than the liquid present in the detection area during detection.

Moreover, the device's light source can encompass various technologies such as LED, laser, gas discharge lamps, arc lamps or halogen lamps, each chosen based on their emission characteristics that are suitable for detecting biological or chemical signals. The light source illuminates the detection area, prompting any bound biomarker or ligand to potentially generate a detectable signal through fluorescence, absorption, or other light-related phenomena. In one embodiment the angular intensity distribution of the excitation light is controlled so that the light hits the interface of the side panel and the fluid in the detection area, in an angle that lower than the critical angle so that it undergoes total internal reflection.

The device is crafted to offer a compact and efficient solution for fluid analysis, making it applicable for diverse situations ranging from clinical diagnostics to environmental testing. Its integration of microfluidic manipulation, targeted functionalization, precise optical detection, and adaptable illumination sources enhances its usability across different fields.

The device encompasses a cartridge within the digital microfluidics platform, which is configured to enhance fluid manipulation and detection capabilities. This cartridge includes a first panel made from transparent material. The transparency of the panel facilitates optical components such as lenses or objectives to detect signals from the detection area with minimal obstruction.

In a preferred embodiment the DMF platform has a first panel made of a transparent material that is conductive or is made conductive.

The first panel can be constructed from a transparent dielectric material to be inherently conductive, or a non-conductive material can be coated by conductive substances such as indium tin oxide (ITO), fluorine doped tin oxide (FTO), aluminum-doped zinc oxide (AZO) gold or other conductive substances known to a person skilled in the art to obtain a transparent conductive panel. The transparent dielectric material is preferably selected from inherently conductive polymers such as polyacetylene, polyphenylene vinylene, polypyrrole, polythiophene, polyaniline, polyphenylene sulfide, or other polymers known to a person skilled in the art.

Conductivity assists in digital microfluidics, enabling control of droplet movement across the panel through electric fields. By incorporating conductive coatings on transparent materials, the device may ensure that activities such as fluid dispensing, movement, mixing, and detection can be effectively attainable.

The choice of coating material can be tailored based on specific requirements such as transparency levels, conductivity efficiency, or the stability of the coating under various operational conditions. For instance, ITO and FTO are associated with excellent conductive properties and high transparency, making them suitable for many optical applications. Aluminum-doped zinc oxide and gold layers provide alternative conductive solutions, each offering beneficial attributes in terms of conductivity and mechanical durability.

This feature renders the device particularly adjustable to environments where optical clarity is advantageous, such as biomedical imaging, while also permitting microfluidic operations through electrically controlled droplet dynamics.

Within the device, the digital microfluidics platform includes a cartridge with a second panel positioned opposite the first panel. The design choice for the second panel offers flexibility regarding material transparency and conductivity, allowing it to align with specific detection and electrical manipulation preferences.

In a further preferred embodiment, the DMF platform has thus a second panel opposite of the first one, wherein the panel can be either transparent or nontransparent and is conductive. The second panel can be constructed from a transparent or nontransparent conductive dielectric material to be inherently conductive. The design choice for the second panel offers flexibility regarding material transparency and conductivity, allowing it to align with specific detection and electrical manipulation preferences. For applications where dual-side optical access may be beneficial, a transparent second panel can enhance light passage, supporting effective signal detection from both sides of the cartridge. Alternatively, when transparency is not of primary significance, a non-transparent panel can be preferable, offering structural integrity and potentially mitigating external interference with optical pathways or detection processes. The material of the second panel can be selected from the same material as the first panel.

The arrangement of conductive panels facing each other enables effective droplet operations influenced by electric forces across the panel surfaces. This capacity benefits fluid handling tasks such as splitting, merging, or routing, enhancing the accuracy and efficiency of the fluid analysis intended by the device.

In another embodiment, the two opposite panels are mechanically linked or bonded together with a defined gap in between. The perimeter around this gap between the panels is sealed, forming a closed internal chamber or cavity within the cartridge. The sealing may be permanent or reversible, depending on application requirements.

In a preferred embodiment, the internal fluid management within the cartridge may be enhanced by connecting the cartridge interior to external fluid containers or reservoirs via integrated tubing or channels. These external fluid connections allow the introduction or the removal of fluid reagents, buffer solutions, or sample materials from outside the cartridge.

Collectively, these attributes contribute to the adaptability of the device, offering potential utility across scientific, medical, or industrial fields where precise microfluidic control and reliable detection capabilities may be advantageous.

In another preferred embodiment, at least one of the panels of the cartridge can be modified such that the conductive area is divided into smaller areas that can be electrically activated independently from each other. This division into smaller areas allows for precise control and movement of droplets across defined paths or regions within the cartridge.

In the context of the device, modifications to at least one panel of the cartridge within the digital microfluidics platform are facilitated to enhance its functional versatility. These optional modifications can divide the conductive area of the panel into smaller, discrete sections that may each be electrically activated independently. This partitioning offers improved control over fluid movement in more localized domains, supporting operational flexibility in fluid manipulation tasks.

This division of conductive areas can provide the capability for independent electrical activation, enabling the creation of programmable pathways for fluid droplets on the panel surface. With segmented conductive regions, the device may implement complex fluid handling protocols like transporting, mixing, analyzing, or reacting distinct droplets in parallel or sequentially.

The option for independent control over each section may be offered through microcontrollers or electronic circuits that selectively apply voltage to selected segments of the panel, thus modulating droplet behavior in detailed zones. Such a setup can be advantageous for assays or experiments focusing on precision and reproducibility, where individual droplet trajectories and interactions have value for accurate measurement or reaction processes.

Moreover, this configuration may permit simultaneous processing of multiple samples, enabling high-throughput methodologies for diagnostic applications, or allows for a dynamic adaptability as particular areas can become active or inactive based on real-time needs of the experiment or analysis.

The ability to electronically activate and deactivate individual segments can streamline operations, minimize cross-contamination, and optimize reagent utilization, making the device suitable for a range of applications demanding agility and versatility in fluid handling. Therefore, partitioning conductive areas into independently activatable segments may improve the patent application's capability, offering customizable and efficient fluid dynamics advantageous in diverse disciplines.

In another preferred embodiment, at least one panel of the cartridge is coated with an antifouling agent. This prevents or minimizes the nonspecific adsorption of biomolecules or contaminants to the panels, thereby supporting the operational integrity and reliability of fluid manipulations.

Antifouling agents included in coatings can lessen the likelihood for biological materials, chemical residues, or particulates to adhere to the panel surface, which could interfere with optical detection or fluid dynamics. By addressing surface fouling, the coating facilitates consistent electrical activation and fluid movement across the panel, contributing to reproducible and accurate assay performance.

Suitable antifouling agents might include non-sticky polymers, hydrophobic molecules, or other materials designed to resist contaminant adhesion and maintain chemical stability under the device's operational conditions. The selection of antifouling agent can be adjusted based on the application, environmental conditions, and specific fluid characteristics involved in the device's operational scope. In yet another preferred embodiment, the antifouling agent is selected from polyethylene glycol (PEG), a PEG-derivative, a hydrogel, a nanoporous metal surface, bovine serum albumin, carbon nanotube, a lipid bilayer, poloxamer or dextran. The term derivative is defined as a compound that can be derived from a similar compound by a chemical reaction. In the present invention a PEG-derivative can thus be interpreted as every compound obtained by a conjugation reaction of PEG with proteins, lipids or chemical molecules.

Polyethylene glycol (PEG) or PEG-derivatives present a potential choice due to their hydrophilic properties and capability to resist protein adsorption, making them suitable for maintaining clean panel surfaces. Hydrogels, comprising polymeric networks capable of retaining substantial amounts of water relative to their dry weight, offer an additional layer that can repel fluids while maintaining a stable, non-adhesive surface.

Nanoporous metal surfaces provide a mechanical means against adherence while allowing for optical transparency and electrical conductivity, depending on their configuration. Bovine serum albumin (BSA), known for its function as a blocking agent, might help reduce nonspecific interactions, thereby enhancing the panel's operational quality.

Carbon nanotube coatings could offer a robust non-stick surface due to their structural characteristics, and lipid bilayers introduce a biologically inspired antifouling method, utilizing selective permeability and non-adhesive properties. Poloxamers, with their surfactant nature, might effectively create a barrier against adhesion by modifying surface tension. Dextran, a polysaccharide, imparts hydrophilic qualities that may reduce fouling while ensuring biological compatibility.

The selection among these agents can be influenced by the specific requirements of the fluid being analyzed, the operational environment, and the desired duration and effectiveness of the antifouling properties. Introducing such agents aims to improve the device's performance by decreasing maintenance needs and enhancing the reliability of biomarker or ligand detection across various applications, thus serving as a beneficial tool in analytical and diagnostic contexts.

In another preferred embodiment, at least one of the panels comprises a detection area wherein the detection area is functionalized with a ligand, an antibody, an aptamer, a protein, a peptide and/or an oligonucleotide comprising DNA, RNA, locked nucleic acids (LNA), or any combination thereof, including chimeric sequences composed of mixtures of natural and/or synthetic nucleic acid analogues. This functionalization enables the binding and subsequent detection and/or quantification of a biomarker or a ligand in a fluid.

In the device, at least one of the panels of the cartridge may encompass a specially defined detection area, which can be functionalized to operate as an interactive surface for assessing the presence of biomarkers or ligands. Such functionalization involves optionally attaching recognition molecules to the panel surface, potentially allowing specific binding interactions with target analytes present in the fluid being examined. The recognition molecules may be selected from a broad range, including ligands, antibodies, aptamers, proteins, peptides, and oligonucleotides. These selections are based on their potential to bind with specificity and affinity to chosen biomarkers or ligands, improving the device's capability to sensitively detect specific substances.

In another preferred embodiment, the detection area comprises at least two separate sections functionalized with two different ligands, antibodies, aptamers, proteins, peptides and/or oligonucleotides comprising DNA, RNA, locked nucleic acids (LNA), or any combination thereof, including chimeric sequences composed of mixtures of natural and/or synthetic nucleic acid analogues to detect the at least two biomarkers or at least two ligands. The presence of separate sections each containing a different functionalization allows for the simultaneous detection of more than one biomarker and/or ligand at the same time.

Oligonucleotides might include DNA, RNA, locked nucleic acids, or any combination thereof. Chimeric sequences may also be contemplated, permitting mixtures of natural and synthetic nucleic acid analogues to address diverse analytical requirements. Chimeric sequences feature alternating units that might provide enhanced binding characteristics or stability across various environmental conditions.

The functionalization with these molecules may transform the detection area into a dynamic platform, suitable for capturing specific biomarkers or ligands. This configuration enables the conversion of biomolecular interactions into detectable signals, which can then be measured using the optical components possibly integrated into the device. This arrangement can be suitable for detecting diverse biological entities, accommodating a range of diagnostic or research applications, including clinical testing, environmental monitoring, and biosecurity assessments. The aim is to offer a detection capability that might be precise, reliable, and rapid, employing the specificity and affinity attributes of various functionalized molecules. Consequently, this may support the device's performance across situations necessitating accuracy and throughput, making it a versatile tool in biological and chemical analysis.

In another preferred embodiment, the at least one of the detection areas can be illuminated by total internal reflection fluorescence (TIRF), wherein the light can be injected through the side of the transparent panel, through a prism that is connected to the transparent panel, or through a free formed optical surface that is attached to or integrated in the transparent panel, the free formed surface being configured such that light entering from outside the cartridge is directed at an angle that induces total internal reflection at the inner interface of the transparent panel, thereby generating an evanescent field for selective excitation within the detection area. A free formed optical surface is a customdesigned optical component with irregular surfaces for precise light manipulation. Unlike traditional, symmetrical lenses, freeform optics offer unique benefits: Compactness and weight reduction: By combining multiple elements into one, freeform optics enable miniaturized devices. By using these free form optics, the size of the cartridge and subsequently the size of the device can be drastically reduced. The illumination may be modulated or pulsed in synchronization with imaging device exposure to optimize signal-to-noise ratios and imaging precision.

TIRF offers targeted excitation and subsequent fluorescence detection, improving signal quality. The configuration may be adaptable to allow light to be injected through alternative arrangements associated with the transparent panel to optimize the total internal reflection mechanism. TIRF enables detection directly at the surface thereby detecting the interaction with the fluid and the coating of the at least one panel. This reaction is fast and thus, the detection is fast as well. There is no need that a reaction takes place throughout the whole volume of a sample, as it is for example the case for ELISA analysis. Therefore, this preferred embodiment allows for a fast but at the same time highly sensitive analysis of a fluid.

One possible approach involves directing light through the side of the transparent panel. The panel's material and design can facilitate guiding light at an angle suitable for achieving total internal reflection across its inner interface. This action can develop an evanescent field, enabling selective excitation within the designated detection area and the capability to detect fluorescence signals from specific biomarker or ligand interactions.

Another arrangement might employ a prism to achieve the desired light angle. The prism is optionally connectable to the transparent panel, allowing precise light injection at an appropriate angle until total internal reflection conditions are attained, similarly resulting in an evanescent wave across the interface.

In yet another preferred embodiment the lens or objective positioned to detect the signal of the at least one detection area is selected from an objective lens, microscope optics, spectral filtering, or dispersive elements. The lens or objective positioned to collect emitted fluorescent signals from the detection area. Emitted signals can be spectrally filtered or analyzed prior to detection. Furthermore, the lens or objective is preferably positioned on the opposite side of the panel comprising the detection area.

In another preferred embodiment the lens or objective detects and analyzes the emitted fluorescence of the TIRF illumination by the use of spatially resolved detectors. Imaging and signal acquisition is performed using spatially resolved detectors, such as CCD or CMOS sensors, effectively forming an integrated microscopy platform capable of fluorescence-based detection and analysis. This setup is designed to facilitate the capture of fluorescence signals generated at the detection area's surface, allowing for detailed analysis and increased sensitivity in biomarker or ligand identification. The resolution offered by these detectors enhances the ability to discern spatial variations in signal intensity, which can be beneficial in differentiating between target-specific binding events and non-specific background signals. The spatially resolved detector can be operatively connected to a computing unit or control system configured to process and analyse the detected signals. The analysis results can be used in real time or near-real time to determine subsequent experimental steps, such as droplet movement, reagent addition, mixing, dilution, the duration of the detection period, temperature changes, or initiation of further detection cycles, thereby enabling adaptive and automated assay workflows within the cartridge. The analysis can include predefined physical, chemical, or thermodynamic models, or utilize data-driven approaches such as machine learning or artificial intelligence algorithms.

When used with the lens or objective, the spatially resolved detectors may be optimized for improved focus and sharpness due to the optical setup aiming to minimize aberrations and optimize light collection. This pairing supports mapping of fluorescent outputs across the detection area, providing high-resolution images that can be analyzed for quantitative or qualitative assessments.

In another preferred embodiment, the cartridge further comprises one or more separate working areas, wherein these separate working areas can be designed to be used for mixing, incubating, reagent activation, pre-treatments or other steps of an assay protocol. These separate working areas can be included into the cartridge to perform complete assay protocols within the DMF platform of the present invention including all pre-treatment and potential reaction steps. Performing the whole assay on a microscale reduces the amount of reactants necessary, thereby increasing the feasibility and economy of these assays. This setup provides procedural adaptability within a single cartridge, helping streamline workflow and potentially improve assay efficiency.

A mixing section can for example be configured to enable effective blending of reagents and samples, promoting homogeneity and enhancing reaction conditions within assay protocols. Mixing is achievable through automated systems involving electronic actuation, ensuring consistency and speed.

Incubation zones may offer controlled conditions for nurturing biochemical reactions or allowing time-dependent processes to progress effectively. These zones could feature specific temperature controls to refine conditions for the reaction kinetics or biological activities intended for observation. The incubation zones can be used to initiate or sustain one or more reactions, wherein reaction products or intermediates are periodically or aperiodically transferred to the detection area to determine the active concentration of the at least one biomarker and/or the at least one ligand.

Designated sections for reagent activation can prepare reagents required for detection or analysis, using mechanisms that administer energy or chemical treatments to initiate reagent functionality.

Pre-treatment areas address preparatory steps that may include sample purification, concentration adjustments, or other modifications beneficial for assay precision and reliability. This step can help ensure samples are well-prepared for downstream processes, contributing to assay integrity.

In another preferred embodiment, the cartridge is equipped with at least one thermally conductive element. By incorporating thermally conductive elements into the cartridge, every step of the assay can be controlled thermally to ensure fast and efficient treatment steps, reaction steps or efficient binding of the biomarker or the ligand to the respective binding moiety present in the functionalized detection area.

In yet another preferred embodiment, the at least one thermally conductive element can either be embedded between the two opposite panels of the cartridge or by thermal contact to the outside of at least one of the panels. These temperature control elements enable localized heating or cooling of selected regions, allowing dynamic temperature regulation of droplets or sample volumes during processing.

This modular thermal control enables protocols requiring specific temperature profiles, such as enzymatic reactions, denaturation/annealing cycles, or accelerated binding steps, to be executed within the cartridge in a spatially controlled and energy-efficient manner.

Materials selected for the thermally conductive element might include metals like copper or aluminum, recognized for their thermal conductivity and mechanical stability. Alternatively, specially tailored polymers or composites could be appropriate when customized for microfluidic applications, combining conductive properties with seamless integration into the cartridge design.

The problem underlying the present invention is further solved by a method for detecting and/or quantifying at least one biomarker comprising the steps:
I) Isolating/preparing a fluid comprising at least one biomarker or at least one ligand.
II) Injecting said fluid into the DMF of the device according to any one of claims 1 to 14.
III) Detection and/or quantification of the at least one biomarker or at least one ligand contained in the fluid.

The described method outlines a process for detecting and quantifying at least one biomarker, utilizing the functionality of a device equipped with digital microfluidics. This method includes a sequence of steps that guide the analytical procedure from sample preparation through to detection and quantification.

Firstly, the method may involve isolating or preparing a fluid that contains the biomarker or ligand of interest. This preparation step ensures that the fluid is suitably treated or modified to be compatible with microfluidic operations and detection systems. Preparation might include isolation, dissolution, filtration, extraction, purification, concentration, or stabilization processes, depending on the nature of the sample and the biomarkers or ligands under investigation.

Once the fluid is prepared, the next step might involve injecting it into the DMF platform of the device. The digital microfluidics system provides a regulated environment for fluid manipulation, allowing precise handling and delivery of the sample to designated detection areas within the cartridge. This step utilizes the capabilities of DMF to position the sample optimally for subsequent analysis.

The final step of the method focuses on the detection and/or quantification of the biomarker or ligand present in the fluid. Using functionalities provided by the device, such as optical detection systems or functionalized surfaces in detection areas, this step captures, visualizes, and measures signals corresponding to biomarker interactions. The device may employ techniques like fluorescence, absorbance, or other optical signal analyses to identify the presence and quantity of the target molecules.

This method offers potential benefits in terms of efficiency, precision, and adaptability. By integrating sample preparation, fluid handling, and analytical detection within a cohesive framework, it enhances workflows across biomedical research, clinical diagnostics, and other scientific applications where biomarker quantification is advantageous.

It is to be noted that each and every embodiment as disclosed in the present application can be combined with each other without any limitation. Further, embodiments disclosed for the device are applicable to the method and vice versa.

## Claims

1. A device to detect and/or quantify at least one biomarker or at least one ligand in a fluid, comprising a digital microfluidics (DMF) platform, at least one detection area, a lens or an objective, and a light source, **characterized in that**
the DMF platform comprises a cartridge with two opposite panels, whereas both panels are conductive or made conductive, and
in the detection area at least one of the panels can be functionalized to detect the at least one biomarker and,
a lens or an objective is positioned to detect the signal of the at least one detection areas and,
the light source is selected from LED, laser, gas discharge lamps, arc lamp, or halogen lamps.

2. A device according to claim 1, wherein the cartridge of the DMF platform has a first panel made of a transparent material that is conductive or is made conductive through coating with indium tin oxide (ITO), fluorine doped tin oxide (FTO), aluminum-doped zinc oxide (AZO) gold or other conductive substances.

3. A device according to claims 1 or 2, wherein the cartridge of the DMF platform has a second panel opposite of the first one, wherein the panel can be transparent or nontransparent and is conductive.

4. A device according to any one of claims 1 to 3, wherein at least one panel of the cartridge can be modified such that the conductive area is divided into smaller areas that can be electrically activated independently from each other.

5. A device according to any one of claims 1 to 4, wherein at least one panel of the cartridge is coated with an antifouling agent.

6. A device according to any one of claims 1 to 5, wherein the antifouling agent is a PEG, a PEG-derivative, a hydrogel, a nanoporous metal surface, bovine serum albumin, carbon nanotube, a lipid bilayer, poloxamer or dextran.

7. A device according to any one of claims 1 to 6, wherein at least one of the panels comprises a detection area, wherein the detection area is functionalized with a ligand, an antibody, an aptamer, a protein, a peptide and/or a oligonucleotide comprising DNA, RNA, locked nucleic acids (LNA), or any combination thereof, including chimeric sequences composed of mixtures of natural and/or synthetic nucleic acid analogues to detect the at least one biomarker or at least one ligand.

8. A device according to claim 7, wherein the detection area comprises at least two separate sections functionalized with two different ligands, antibodies, aptamers, proteins, peptides and/or oligonucleotides comprising DNA, RNA, locked nucleic acids (LNA), or any combination thereof, including chimeric sequences composed of mixtures of natural and/or synthetic nucleic acid analogues to detect the at least two biomarkers or at least two ligands.

9. A device according to any one of claims 1 to 8, wherein the at least one of the detection areas can be illuminated by total internal reflection fluorescence (TIRF), wherein the light can be injected through the side of the transparent panel, through a prism that is connected to the transparent panel, or through a free formed optical surface that is attached to or integrated in the transparent panel, the free formed surface being configured such that light entering from outside the cartridge is directed at an angle that induces total internal reflection at the inner interface of the transparent panel, thereby generating an evanescent field for selective excitation within the detection area.

10. A device according to any one of claims 1 to 9, wherein the lens or objective positioned to detect the signal of the at least one detection area is selected from an objective lens, microscope optics, spectral filtering, or dispersive elements.

11. A device according to claims 9 or 10, wherein the lens or objective detects and analyzes the emitted fluorescence of the TIRF illumination by the use of spatially resolved detectors.

12. A device according to any one of claims 1 to 11, wherein the cartridge further comprises one or more separate working areas, wherein these separate working areas can be designed to be used for mixing, incubating, reagent activation, pre-treatments or other steps of an assay protocol.

13. A device according to any one of claims 1 to 12, wherein the cartridge is equipped with at least one thermally conductive element.

14. A device according to any one of claims 1 to 13, wherein the at least one thermally conductive element can either be embedded between the two opposite panels of the cartridge or by thermal contact to the outside of at least one of the panels.

15. Method for detecting and/or quantifying at least one biomarker comprising the steps:
I) Isolating/preparing a fluid comprising at least one biomarker or at least one ligand.
II) Injecting said fluid into the DMF of the device according to any one of claims 1 to 14.
III) Detection and/or quantification of the at least one biomarker or at least one ligand contained in the fluid.
